# FASCICULE DE BREVET EUROPEEN

(11) **EP 1 258 226 B1**
(45) Date de publication et mention de la délivrance du brevet: **06.08.2008**
(21) Numéro de dépôt: 01870104.5
(22) Date de dépôt: 16.05.2001
(51) Int. Cl.: A61B 18/02

(54) **Instruments destiné aux traitements cryogeéniques dans le secteur médical, paramédical et cosmétique**
Vorrichtung zur medizinischen, paramedizinischen oder kosmetischen Kältebehandlung
Apparatus for medical, paramedical or cosmetic cryogenic treatment

(43) Date de publication de la demande: 20.11.2002
(73) Titulaire: N.V. H&O Equipments, 9320 Erembodegem (BE)
(72) Inventeur: Hermans, Erik, 1570 Vollezele (BE)
(74) Mandataire: pronovem

(56) Documents cités:
- US-A- 3 951 152
- US-A- 4 336 691
- US-A- 4 376 376
- US-A- 5 330 745
- US-A- 6 141 985

## Description

### Objet de l'invention

La présente invention concerne un instrument convenant à divers traitements cryogéniques, tant pour le secteur médical ou paramédical, que pour le secteur cosmétique.

### Arrière-plan technologique

De nombreuses affections dermatologiques sont actuellement traitées par voie cryogénique en utilisant un "jet" de gaz, généralement de N₂O à très basse température. Cette technique cryogénique est également utilisée dans d'autres disciplines médicales et dans des secteurs cosmétiques.

On utilise entre autres à cet effet un instrument, dans lequel on introduit une cartouche de gaz liquéfié par exemple N₂O qui est mis en communication, généralement par perforation d'une membrane ou d'un opercule, avec un micro-applicateur se présentant sous forme d'une aiguille pourvue d'un alésage de très faible diamètre, l'ensemble étant contenu dans une gaine tenue à la main par l'opérateur.

D'autres gaz que le N₂O (gaz hilarant) peuvent bien entendu être utilisés.

L'expansion du gaz contenu à l'état liquide dans la cartouche sous la forme gazeuse, se traduit par la libération d'un "jet" à très basse température (de l'ordre de -28 à -90°C).

Le jet est essentiellement constitué de N₂O, en partie à l'état gazeux et en partie à l'état liquide.

Deux difficultés majeures et quasiment prohibitives se présentent lors de la mise en oeuvre de cette technique.

Il est apparu en effet que des impuretés, essentiellement solides, sont également présentes dans le jet et qu'elles provoquent rapidement un colmatage du micro-applicateur.

La Demanderesse a observé que ces impuretés sont occasionnées essentiellement par des résidus qui soit proviennent des solvants utilisés lors du nettoyage préalable de la cartouche, soit sont des particules libérées lors du perçage de la membrane ou de l'opercule prévu à cet effet sur la cartouche.

D'autres impuretés peuvent provenir par exemple des frottements entre des parties du corps de l'instrument entre elles ou avec la cartouche, lors de la mise en place de cette dernière.

Il n'est pas exclu que le processus de production du gaz liquéfié soit également une cause complémentaire des obstructions observées par suite de la présence d'impuretés dans le gaz lors de son conditionnement en cartouches.

Même des particules de très petites dimensions peuvent être la cause d'obstructions importantes compte tenu de la très petite dimension de l'alésage pratiqué dans le micro-applicateur et de l'effet de "nucléation" auquel il sera fait référence ci-après.

Un autre phénomène que l'on observe est la condensation, sous forme de glace, de l'humidité contenue dans l'air atmosphérique qui contribue fortement au colmatage, en fonction de la nature du matériau utilisé pour la fabrication du micro-applicateur, par suite d'un phénomène de "givrage".

Ce phénomène de "givrage" s'observe tout particulièrement par exemple pour des micro-applicateurs métalliques. Il semble également que la présence de particules d'impuretés facilite le "givrage" par un effet de nucléation, c'est-à-dire la condensation sous forme de glace de l'air atmosphérique sur les petites particules éventuellement présentes dans le jet "gazeux".

### But de l'invention

La présente invention vise à éviter les inconvénients décrits des instruments selon l'état de la technique, en particulier pour éviter leur colmatage et permettre ainsi de meilleures performances en facilitant leur utilisation.

### Eléments caractéristiques de l'invention

La Demanderesse a observé que les conditions opératoires optimales reposent sur le principe qu'un micro-applicateur doit présenter un alésage de l'ordre de 35 à 80 µm et qu'un écoulement régulier et constant du gaz liquéfié contenu dans la cartouche ne peut être obtenu que si les particules étrangères éventuellement présentes dans le flux du micro-applicateur sont telles que toutes celles supérieures à 3 µm ont été éliminées.

Ceci peut être obtenu en utilisant un gaz condensé ayant subi une épuration préalable pour éliminer les matières solides.

Dans la plupart des cas cependant on observe en pratique que même le recours à du gaz condensé spécialement épuré ne résout pas nécessairement le problème et selon une caractéristique complémentaire de l'invention correspondant à une forme d'exécution préférée, il est prévu de pourvoir le micro-applicateur d'un filtre amovible retenant les particules supérieures à 3 µm, et préférablement supérieures à 0,5 µm.

Des filtres de différentes natures peuvent convenir, tels que des céramiques poreuses, du matériau cellulosique etc.

Pour éviter que le filtre ne se colmate progressivement par accumulation de dépôt de particules lors de l'usage répétitif de plusieurs cartouches successives dans le même appareillage, selon une forme d'exécution particulièrement préférée de l'invention, il est prévu que le filtre soit remplacé à chaque remplacement de la cartouche de gaz. De cette manière, on est assuré que le remplacement d'une cartouche entraîne automatiquement le remplacement du filtre par un nouveau filtre en évitant le colmatage du micro-applicateur.

La Demanderesse s'est également attachée à résoudre les difficultés observées résultant du "givrage", c'est-à-dire le colmatage du micro-applicateur par de la glace provenant de l'humidité atmosphérique. Elle s'est aperçue que le recours à des matériaux, en particulier des matériaux synthétiques tels que le polycarbonate ou une résine du type PEEK réduit fortement ce phénomène dans une mesure telle que les phénomènes éventuels de givrage n'entraînent pas de colmatage.

D'autres matériaux dont les propriétés de conductibilité thermique sont adéquates, tel que le verre, peuvent convenir à cet effet. Le matériau doit bien entendu être choisi de manière à résister à la fois aux très basses températures observées lors des traitements, mais également aux températures élevées nécessaires à la stérilisation.

A titre complémentaire, l'invention vise également à fournir un micro-applicateur à usage unique convenant pour l'instrument de traitement cryogénique tel que décrit.

### Brève description du dessin

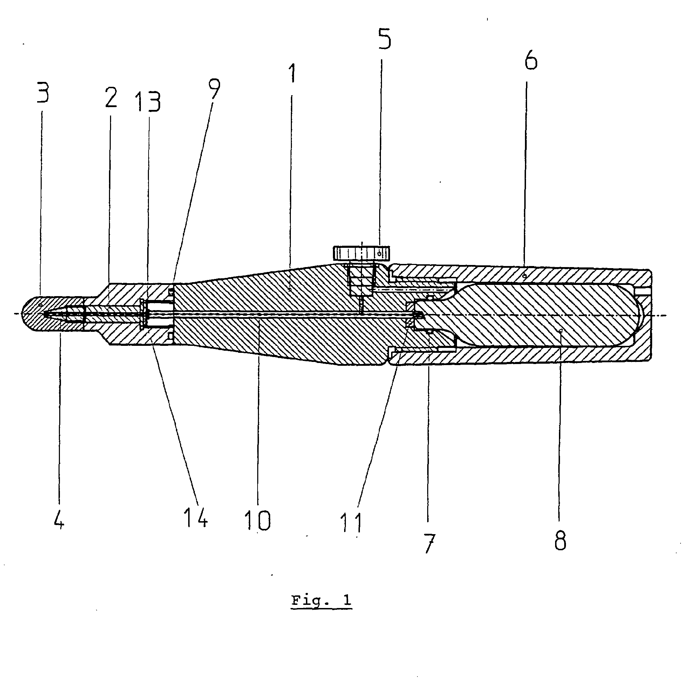

La figure 1 représente une vue en coupe de l'instrument selon l'invention.

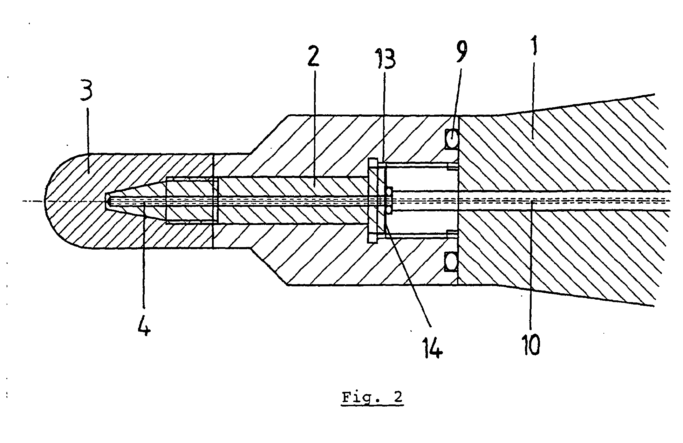

La figure 2 représente une vue en coupe agrandie de la partie avant de cet instrument.

Les mêmes repères de références sont utilisés pour des éléments constitutifs identiques dans les deux figures.

### Description d'une forme d'exécution préférée de l'invention

L'instrument de traitement cryogénique est constitué d'un corps 1. Sur ce corps, un micro-applicateur 2 protégé lorsqu'il n'est pas en usage par un capuchon 3 est fixé de manière étanche mais amovible grâce à un joint torique de type O-ring 9.

Sur la partie arrière du corps 1, une gaine 6 est montée. Elle peut recevoir une cartouche de gaz condensé 8. Un O-ring 7 assure la liaison étanche entre la cartouche et le corps 1. En fonctionnement, lorsque la cartouche 8 est en place, la fixation de la gaine 6 sur le corps 1 provoque le percement d'un opercule 11 obturant la cartouche 8, mettant ainsi en communication par le conduit 10 la cartouche 8 avec le micro-applicateur 2.

Le micro-applicateur 2 comporte un alésage 4 de 35 à 80 µm. Il est relié par un O-ring 13 avec le conduit 10 avec interposition d'un filtre 14 qui est de préférence maintenu en place sur la partie amovible avant, et plus spécifiquement à l'arrière du micro-applicateur 2.

Le réglage de débit est réalisé par une roue à molette 5 ou tout autre dispositif assurant une fonction similaire.

La solution d'un filtre 14 disposé sur le micro-applicateur 2 constitue une solution particulièrement avantageuse puisque le remplacement du filtre 14 est facilement réalisé par démontage du micro-applicateur 2 simultanément avec le démontage de la gaine 6 pour le remplacement de la cartouche 8. Dans ce cas, il n'est pas nécessaire (quoi qu'utile) d'utiliser un gaz condensé particulièrement épuré pour la cartouche 8.

Bien qu'on ait décrit des formes d'exécution particulièrement avantageuses de l'invention, des variantes de constitution peuvent être prévues pour l'appareillage décrit, tout en restant dans le cadre des revendications.

L'invention n'est en particulier pas limitée aux gaz condensés indiqués, ni aux formes et dimensions de l'appareillage proposé.

## Revendications

1. Instrument convenant aux traitements cryogéniques, tant pour le secteur médical ou paramédical que pour le secteur cosmétique, comportant un micro-applicateur (2) présentant un alésage de l'ordre de 35 à 80 µm alimenté par un flux de gaz dans lequel toutes les particules étrangères supérieures à 3µm ont été éliminées.

2. Instrument selon la revendication 1, **caractérisé en ce qu'**il comporte une cartouche contenant un gaz condensé épuré dans lequel les matières solides ont été éliminées.

3. Instrument selon la revendication 1 ou 2, **caractérisé en ce qu'**il comporte une cartouche (8) contenant du N₂O

4. Instrument selon l'une quelconque des revendications 1 à 3, **caractérisé en ce que** le micro-applicateur (2) comporte un filtre (14) retenant les particules supérieures à 3 µm.

5. Instrument selon la revendication 4, **caractérisé en ce que** le micro-applicateur (2) comporte un filtre (14) retenant les particules supérieures à 0,5 µm.

6. Instrument selon la revendication 4 ou 5, **caractérisé en ce que** le filtre (14) est_logé dans /ou sur le micro-applicateur (2).

7. Instrument selon l'une quelconque des revendications 1 à 6, **caractérisé en ce que** le micro-applicateur (2) est constitué par des matériaux synthétiques tels que le polycarbonate ou une résine du type PEEK afin de réduire les phénomènes de givrage et le colmatage de celui-ci.

8. Micro-applicateur (2) pour un instrument selon l'une quelconque des revendications 1 à 7, **caractérisé en ce qu'**il est pourvu d'un filtre amovible monté sur celui-ci.

9. Utilisation de l'instrument selon l'une quelconque des revendications 1 à 7 pour le traitement cosmétique de la peau.

## Claims

1. Apparatus for cryogenic treatments, for use in the medical or paramedical field as well as for the cosmetic field, comprising a microapplicator (2) having a bore diameter of 35 to 80 µm supplied with a gas flow from which all foreign particles bigger than 3 µm have been eliminated.

2. Apparatus according to claim 1 **characterised in that** it comprises a cartridge of purified condensed gas from which all solid materials have been eliminated.

3. Apparatus according to claim 1 or 2 **characterised in that** it comprises a cartridge (8) with N₂O.

4. Apparatus according to any of claims 1 to 3 **characterised in that** the microapplicator (2) comprises a filter (14) arranged to retain particles bigger than 3 µm.

5. Apparatus according to claim 4 **characterised in that** the microapplicator (2) comprises a replaceable filter (14) arranged to retain particles bigger than 0.5 µm .

6. Apparatus according to claim 4 or 5 **characterised in that** the filter (14) is located in or on the microapplicator (2).

7. Apparatus according to any of the claims 1 to 6, **characterised in that** the microapplicator (2) consists of synthetic materials such as the polycarbonate or a resin such as PEEK to reduce the phenomena of icing and the clogging-up of said microapplicator.

8. Microapplicator (2) for an apparatus according to any of the claims 1 to 7, **characterised in that** it comprises a removable filter mounted thereon.

9. Use of the apparatus according to any of the claims 1 to 7 for the cosmetic treatment of the skin.

## Patentansprüche

1. Instrument, das für kryogene Behandlungen geeignet ist, sowohl auf dem medizinischen oder paramedizinischen Sektor als auch auf dem kosmetischen Sektor, umfassend einen Mikroapplikator (2), der eine Bohrung im Bereich von 35 bis 80 µm aufweist, die durch einen Gasstrom gespeist wird, aus welchem alle Fremdkörper über 3 µm entfernt worden sind.

2. Instrument nach Anspruch 1, **dadurch gekennzeichnet, dass** es eine Kartusche umfasst, die ein gereinigtes kondensiertes Gas enthält, aus dem die Feststoffe entfernt worden sind.

3. Instrument nach Anspruch 1 oder 2, **dadurch gekennzeichnet, dass** es eine Kartusche (8) umfasst, die N₂O enthält.

4. Instrument nach einem der Ansprüche 1 bis 3, **dadurch gekennzeichnet, dass** der Mikroapplikator (2) einen Filter (14) umfasst, der die Körper über 3 µm zurückhält.

5. Instrument nach Anspruch 4, **dadurch gekennzeichnet, dass** der Mikroapplikator (2) einen Filter (14) umfasst, der die Körper über 0,5 µm zurückhält.

6. Instrument nach Anspruch 4 oder 5, **dadurch gekennzeichnet, dass** der Filter (14) in/oder auf dem Mikroapplikator (2) sitzt.

7. Instrument nach einem der Ansprüche 1 bis 6, **dadurch gekennzeichnet, dass** der Mikroapplikator (2) aus synthetischen Materialien, wie etwa Polykarbonat oder einem Harz der Art PEEK gebildet ist, um die Vereisungs- und Verstopfungsphänomene dieses zu reduzieren.

8. Mikroapplikator (2) für ein Instrument nach einem der Ansprüche 1 bis 7, **dadurch gekennzeichnet, dass** er mit einem abnehmbaren Filter versehen ist, der auf diesem befestigt ist.

9. Benutzung des Instruments nach einem der Ansprüche 1 bis 7 zur kosmetischen Behandlung der Haut.
